Europäisches Patentamt

European Patent Office

Office européen des brevets

(19)

(11) Veröffentlichungsnummer: **0 392 413**
**A2**

(12) **EUROPÄISCHE PATENTANMELDUNG**

(21) Anmeldenummer: **90106740.5**

(22) Anmeldetag: **09.04.90**

(51) Int. Cl.5: **C12N 15/26, C12P 21/02**

---

(30) Priorität: **12.04.89 DE 3912020**

(43) Veröffentlichungstag der Anmeldung:
**17.10.90 Patentblatt 90/42**

(84) Benannte Vertragsstaaten:
**BE CH DE DK FR GB IT LI NL SE**

(71) Anmelder: **Gesellschaft für Biotechnologische Forschung mbH (GBF)**
**Mascheroder Weg 1**
**D-3300 Braunschweig-Stöckheim(DE)**

(72) Erfinder: **Weigel, Ulrich, Dr.**
**Koellikerstrasse 2**
**D-8700 Würzburg(DE)**
Erfinder: **Meyer, Marita, Dr.**
**Koellikerstrasse 2**
**D-8700 Würzburg(DE)**
Erfinder: **Sebald, Walter, Prof.Dr.**
**Koellikerstrasse 2**
**D-8700 Würzburg(DE)**

(74) Vertreter: **Boeters, Hans Dietrich, Dr. et al**
**Bereiteranger 15**
**D-8000 München 90(DE)**

---

(54) **Verfahren zur Herstellung einer hIL-2-Mutante und hIL-Mutante.**

(57) Die Erfindung betrifft ein Verfahren zur Herstellung einer nach Expression leicht renaturierbaren Mutante des humanen Interleukin-2-Wildtyps sowie diese Mutante.

EP 0 392 413 A2

## Verfahren zur Herstellung einer hIL-2-Mutante und hIL-2-Mutante

hIL-2 läßt sich als rekombinantes Protein (rhIL-2) gentechnologisch in E. coli erzeugen. Die dabei gebildeten Einschlußkörper lassen sich jedoch nur unbefriedigend solubilisieren oder renaturieren.

Erfindungsgemäß wurde nun ein Verfahren konzipiert, mit dem sich eine nach Expression leicht renaturierbares Mutantenprotein des hIL-2-Wildtpys produzieren läßt. Dazu wird
- cDNA, die einen DNA-Bereich umfaßt, der den maturen Bereich von hIL-2 kodiert, einer gezielten Oligonucleotid-Mutagenese (site-directed-mutagenesis) derart unterworfen, daß im hIL-2-Segment D im Bereich der position 94 Lys statt Leu exprimiert wird,
- der DNA-Bereich, der den mutierten maturen Bereich von hIL-2 kodiert, in einen Expressionsvektor integriert,
- der gebildete Hybrid-Vektor in E. coli eingesetzt und
- das hIL-2-Mutantenprotein exprimiert und gegebenenfalls isoliert.

Zur Beschaffung von cDNA, die einen DNA-Bereich umfaßt, der den maturen Bereich von hIL-2 kodiert, oder die den maturen Bereich von hIL-2 kodiert, wird auf Taniguchi et al. (Nature, 302 (1983) 305 - 310) und Devos et al. (Nucleic Acids Research, 11 (1983) 4307 - 4323) und die darin angeführte Literatur verwiesen. Im vorliegenden Zusammenhang werden unter "cDNA, die den maturen Bereich von hIL-2 kodiert" auch cDNAs verstanden, die bei etwa gleicher Anzahl von Basenpaaren Mutanten der konkret im genannten Stand der Technik angegebenen cDNA darstellen, sofern die damit vorzusehenden hIL-2-Muteine gleichfalls in hoher Ausbeute renaturierbar sind.

Hinsichtlich der Durchnumerierung des den maturen Bereich von hIL-2 kodierenden DNA-Bereichs wird hier Devos et al. (loc. cit. 4315 Fig. 3) gefolgt. Für die Bezeichnung der α-helikalen Segmente siehe Brandhuber et al. (Science, 238 (1987) 1707-1709).

Die site-directed-mutagenesis kann gemäß Kramer et al. durchgeführt werden (Nucleic Acids Research, 12 (1984) 9441 - 9455; Cell, 38 (1984) 879 - 887; und Boehringer-Mannheim-Prospekt, Biochemicals for Molecular Biology, (1987) 35 etc.). Das zur Mutagenese verwendete Oligonucleotid kann etwa 6 bis 10 Basen vor und etwa 6 bis 10 Basen hinter der (den) zu ändernden Base(n) enthalten.

Der Fachmann ist auch mit dem Herausschneiden des den mutierten maturen Bereich von hIL-2 kodierenden DNA-Bereichs aus dem Vektor, dem Überführen in einen Expressionsvektor, dem Einsetzen in E. coli sowie dem Exprimieren des hIL-2-Mutantenproteins und ihrer fakultativen Isolierung vertraut (McCarthy et al., Gene 41 (1986) 201 - 206; Kato et al., Biochem. Biophys. Res. Commun., 130 (1985) 692 - 699).

Gemäß einer speziellen Ausführungsform des erfindungsgemäßen Verfahrens baut man in die cDNA, die den DNA-Bereich umfaßt, der den maturen Bereich von hIL-2 kodiert, DNA für ein Initiator-Methionin ein, bevor man die gezielte Oligonucleotidmutagenese durchführt.

Gemäß einer weiteren speziellen Ausführungsform kann man den DNA-Bereich, der den mutierten maturen Bereich von hIL-2 kodiert, aus der cDNA-Mutante als Ncol/BamHI-Fragment herausschneiden.

Vorzugsweise verwendet man für die Expression einen temperaturregulierten Expressionsvektor, beispielsweise pILA502 (ohne linksständigen lambda-Promotor und Polylinker) und/oder einen gängigen E.-coli-Stamm als Host, beispielsweise JM103 (recA⁻).

Für pILA502 vergleiche man Schauder et al. (Gene, 52 (1987) 279 - 283). Weitere geeignete Expressionsvektoren lassen sich von Pharmacia beziehen ("Prokaryotic Gene Fusion Vectors"). Der E.-coli-Stamm JM103 läßt sich gleichfalls von Pharmacia beziehen.

Gemäß einer weiteren Ausführungsform betrifft die Erfindung eine hIL-2-Mutante des Wildtyps, bei der im Segment D im Bereich der Position 94 Leu gegen Lys ausgetauscht ist.

Zur Nomenklatur der hIL-2-Segmente sei auf Brandhüber et al. (Science, 238 (1987) 1707 - 1709) verwiesen. Bereich D umfaßt danach die Positionen 83 bis 101. Nach Devos et al. (Nucleic Acids Research, 11 (1983) 4315 Figur 3) steht in diesem Bereich an den Positionen 85, 94 und 96 jeweils Leu. Vorzugsweise ist Leu der Position 94 gegen Lys ausgetauscht.

Nachstehend wird die Erfindung durch zwei Beispiele näher erläutert.

### Beispiel 1

Es wurde cDNA, die den maturen Bereich von hIL-2 und ein Initiator-Methionin kodierte, einer gezielten Oligonucleotid-Mutagenese gemäß Kramer et al. unterworfen. Das synthetische Oligonucleotid umfaßte 6 Basen vor und nach dem auszutauschenden Nucleotid und war mit Hilfe einer DNA-Synthese-Maschine hergestellt worden (Applied Biosystems, Modell 380). Die auszutauschende Stelle war Position 125 im Bereich F (Positionen 117 bis 133). Dabei wurde Cys gegen Thr ausgetauscht. Die erhaltene Mutation wurde durch DNA-Sequenzanalyse einzelsträngiger Bakteriophagen-DNA verifiziert. Die mutierte cDNA wurde als NcoI/BamHI-Fragment aus der doppelsträngigen viralen DNA herausgeschnitten und mit einem temperatur-regulierten Expressionsvektor kombiniert, der pILA502 mit der Ausnahme entsprach, daß der linksständige lambda-Promotor und der Polylinker fehlten. Als Host wurde ein recA-Derivat des E.-coli-Stammes JM103 verwendet. Die integrierte hIL-2-cDNA wurde sequenziert, um die Mutation u bestätigen. Danach wurde der Stamm für die Expression des Muteins eingesetzt.

Bei der Expression zeigte sich, daß die Einschlußkörper des Muteins (T125) nicht befriedigend solubilisiert und renaturiert werden konnten.

Beispiel 2

Es wurde Beispiel 1 mit der Ausnahme wiederholt, daß von der cDNA ausgegangen wurde, bei der Position 125 einer gezielten Oligonucleotidmutagenese unterworfen worden war. In diesem Beispiel wurde nun zusätzlich Position 94 im Bereich D einer gezielten Oligonucleotidmutagenese unterworfen, um im hIL-2-Segment D im Bereich der Position 94 Lys statt Leu zu exprimieren.

Das Mutein (K94/T125) wurde mit einer Endausbeute von etwa 25 % auf Basis des Proteingehalts der Einschlußkörper gewonnen. Durch die Modifizierung der hydrophoben Position Leu-94 zu Lys-94 wird die Ausbeute überraschenderweise um mehr als das Zehnfache erhöht.

**Ansprüche**

1. Verfahren zur Herstellung eines nach Expression in E. coli in hoher Ausbeute renaturierbaren Mutantenproteins (oder Muteins) des hIL-2-Wildtyps, dadurch *gekennzeichnet*, daß man
- cDNA, die einen DNA-Bereich umfaßt, der den maturen Bereich von hIL-2 kodiert, einer gezielten Oligonucleotid-Mutagenese (site-directed-mutagenesis) derart unterwirft, daß im hIL-2-Segment D im Bereich der Position 94 Lys statt Leu exprimiert wird,
- den DNA-Bereich, der den mutierten maturen Bereich von hIL-2 kodiert, in einen Expressionsvektor einsetzt,
- den gebildeten Hybrid-Vektor in E. coli einführt und
- das hIL-2-Mutantenprotein exprimiert und gegebenenfalls isoliert.

2. Verfahren nach Anspruch 1, dadurch *gekennzeichnet*, daß man in die cDNA, die den DNA-Bereich umfaßt, der den maturen Bereich von hIL-2 kodiert, DNA für ein Initiator-Methionin einbaut, bevor man die gezielte Oligonucleotid-Mutagenese durchführt.

3. Verfahren nach Anspruch 1 oder 2, dadurch *gekennzeichnet*, daß man den DNA-Bereich, der den mutierten maturen Bereich von hIL-2 kodiert, aus dem Vektor als NcoI/BamHI-Fragment herausschneidet.

4. Verfahren nach einem der vorhergehenden Ansprüche, dadurch *gekennzeichnet*, daß man einen temperatur-regulierten Expressionsvektor, beispielsweise pILA502 (ohne linksständigen lambda-Promotor und Polylinker) und/oder den modifizierten E. coli-Stamm JM103 (recA⁻) als Host verwendet.

5. hIL-2-Mutantenprotein des Wildtyps, bei der im Segment D im Bereich der Position 94 Leu gegen Lys ausgetauscht ist.

6. hIL-2-Mutantenprotein nach Anspruch 5, dadurch *gekennzeichnet*, daß an der Position 94 Leu gegen Lys ausgetauscht ist.